(19) Europäisches Patentamt
European Patent Office
Office européen des brevets

(11) Publication number: **0 374 919 A1**

(12) # EUROPEAN PATENT APPLICATION

(21) Application number: 89123660.6

(51) Int. Cl.[5]: C07D 489/00, A61K 31/485

(22) Date of filing: 21.12.89

(30) Priority: 21.12.88 JP 322729/88

(43) Date of publication of application: 27.06.90 Bulletin 90/26

(84) Designated Contracting States: AT BE CH DE ES FR GB GR IT LI NL SE

(71) Applicant: Chugai Seiyaku Kabushiki Kaisha
5-1, 5-chome, Ukima Kita-ku
Tokyo(JP)

(72) Inventor: Kanematsu, Ken
5-9-21, Tomogaoka Jonan-ku
Fukuoka-shi Fukuoka-ken(JP)
Inventor: Takayanagi, Issei
2-536-1-1-908, Hazamacho
Funabashi-shi Chiba-ken(JP)
Inventor: Yoshida, Mitsutaka
24-2, Fujimidai
Mishima-shi Shizuoka-ken(JP)

(74) Representative: Vossius & Partner
Siebertstrasse 4 P.O. Box 86 07 67
D-8000 München 86(DE)

(54) **Novel morphine derivative.**

(57) A novel morphine derivative represented by the following general formula:

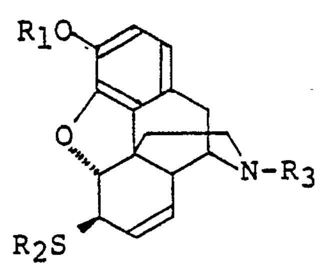

wherein $R_1$ represents a hydrogen atom or a lower alkanoyl group;
$R_2$ represents a lower alkanoyl group; and
$R_3$ represents a cyclopropylmethyl or an allyl group),
and a pharmaceutically acceptable acid addition salt thereof. The compound of the above general formula shows an analgesic activity 5 or more times as high as that of morphine and a narcotic antagonist action. Further, it shows an extremely low drug dependence. These properties make it highly useful as an active ingredient of drugs such as an analgesic or an anesthetic.

EP 0 374 919 A1

# NOVEL MORPHINE DERIVATIVE

This invention relates to a novel 6β-thiomorphine derivative represented by the following general formula (I):

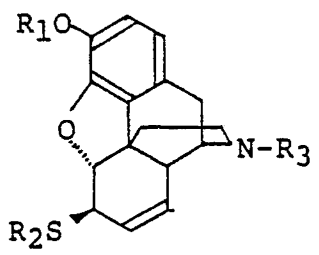

(I)

wherein $R_1$ represents a hydrogen atom or a lower alkanoyl group;
$R_2$ represents a lower alkanoyl group; and
$R_3$ represents a cyclopropylmethyl or an allyl group.

The compound represented by the general formula (I) is expected to be useful as a drug such as a highly effective nonnarcotic analgesic, since it exerts high analgesic and narcotic antagonist actions and yet an extremely low drug dependence.

Morphine, which is known as the major component of opium alkaloids, has been frequently used as drugs such as an anesthetic and an analgesic. However, it is disadvantageous in that it causes drug dependence and liable to cause morphinomania.

It is known that naloxone represented by the following formula (II):

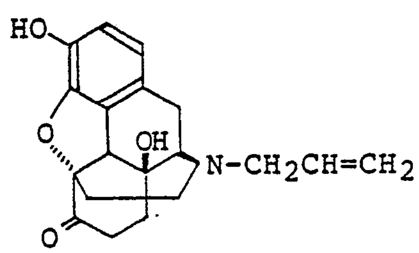

(II)

shows a narcotic antagonist action, similar to the compound of the present invention. However, naloxone has a slight analgesic action, which does not make it so suitable as an analgesic. Thus, it has been urgently required to develop a drug which has potent analgesic and narcotic antagonist actions and yet a low drug dependence.

We have conducted extensive studies in order to overcome the above-mentioned problems. As a result, we have found out that a compound represented by the general formula (I) shows an analgesic action five or more times as high as that of morphine and a narcotic antagonist action, thus achieving the present invention.

Accordingly, the present invention provides a 6β-thiomorphine derivative represented by the general formula (I).

Fig. 1 shows the result of an analgesic examination on the compounds of the present invention and morphine hydrochloride effected by radiant heat-stimulation test using mice.

The 6β-thiomorphine derivative of the present invention, wherein $R_3$ is a cyclopropylmethyl group, may be obtained by, for example, the following process. Namely, cyclopropylmethylnormorphine of the formula (V) is formed from normorphine of the formula (III) by a method reported by Gates and Montzka [M. Gates and T.A. Montzka; J. Med. Chem., 7, 127 (1964)]. Then the product (V) is reacted with $R_2SH$, wherein $R_2$ is as defined above, by Mitsunobu's method [O. Mitsunobu; Synthesis, 1981, 1]. The 6β-thiomorphine derivative of the present invention wherein $R_3$ is an allyl group may be obtained by, for example, the following process. Namely, normorphine represented by the formula (III) is reacted with an allyl bromide and potassium carbonate in a polar solvent, preferably dimethylformamide, to thereby give an N-allylnormorphine. Then the obtained N-allylnormorphine is reacted with $R_2SH$, wherein $R_2$ is as defined above, by Mitsunobu's method.

As the lower alkanoyl groups of $R_1$ and $R_2$ in the compound of the present invention, those carrying 2 to 7 carbon atoms may be used. Preferable examples thereof include acetyl and propionyl groups.

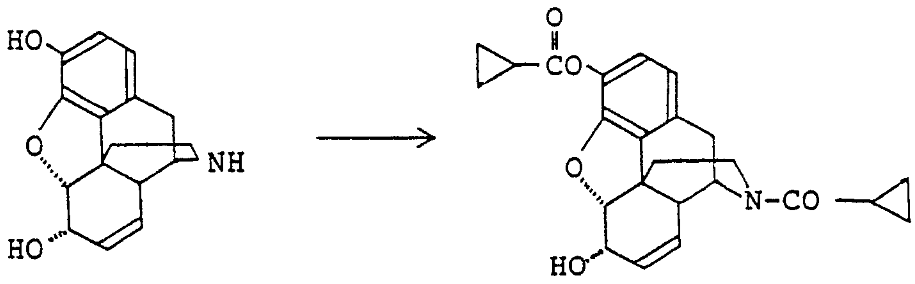

(III) (IV)

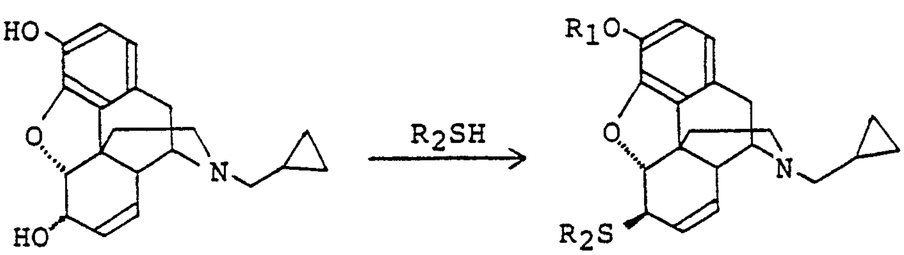


(V)

wherein $R_1$ and $R_2$ are as defined above.

Furthermore, the compound of the general formula (I) may be converted into an acid addition salt, if required. In order to form the acid addition salt for medical application, any pharmaceutically acceptable acid may be used without limitation. Examples of the acid include organic acids such as citric, fumaric, maleic and tartaric acids and mineral acids such as hydrochloric, hydrobromic, nitric and sulfuric acids.

As will be shown in Examples later, the compound of the present invention shows an intense analgesic activity approximately 5 to 6 times as high as that of morphine in a radiant heat-stimulation test using mice. Further, morphine shows scarcely any analgesic activity 3 hours after administration. In contrast thereto, the compound of the present invention still shows a significant analgesic activity, which indicates that it is superior to morphine in prolonged action. Furthermore, the compound of the present invention shows an excellent analgesic action via $\kappa$-receptor and a nonnarcotic action in $\mu$-receptor in a transmural electric stimulation specimen of an extirpated guinea pig ileum piece. These facts suggest that it is highly effective as a drug such as an analgesic.

To further illustrate the present invention, the following Examples will be given.

## Example 1

6.35 ml of diisopropyl azodicarboxylate was added dropwise to 80 ml of a solution containing 8.46 g of triphenylphosphine in dry tetrahydrofuran at 0 °C under a nitrogen gas stream. The obtained mixture was stirred under ice-cooling for 30 minutes. Next, 3.4 ml of thioacetic acid and a suspension of 5.0 g of cyclopropylmethylnormorphine, which had been synthesized from normorphine according to the method reported by Gates and Montzka, in dry tetrahydrofuran were added dropwise thereto under ice-cooling and the mixture was stirred for 4 hours. After distilling off the tetrahydrofuran, the residue was purified by silica gel column chromatography. Thus 3.9 g (yield: 66.2%) of 6$\beta$-acetylthio-N-cyclopropylmethylnormorphine and 1.0 g (yield: 15.3%) of 3-acetyl-6$\beta$-acetylthio-N-cyclopropylmethylnormorphine were obtained in the form of colorless crystals. 2.0 g of the 6$\beta$-acetylthio-N-cyclopropylmethylnormorphine was dissolved in tetrahydrofuran and a hydrogen chloride gas was introduced thereto. After distilling off the tetrahydrofuran, the residue was crystallized from either. Thus 1.9 g of a hydrochloride of the following formula (VI) was obtained in the form of colorless crystals (m.p.: 192 - 194 °C).

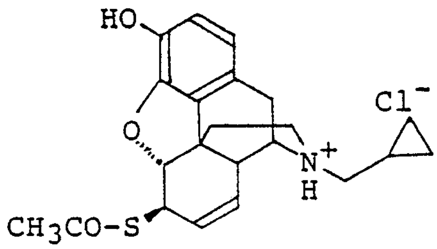

(VI)

Optical rotation $[\alpha]_D^{20}$ : -259.9° ($H_2O$, C = 0.297)
Elemental analysis as $C_{22}H_{25}NO_3S \cdot HCl$
(molecular weight: 419.969)

| | | | |
|---|---|---|---|
| calculated (%): | C 62.92; | H 6.24; | N 3.34 |
| found (%): | C 62.72; | H 6.18; | N 3.14 |

Example 2

1.0 g of the 6β-acetylthio-N-cyclopropylmethylnormorphine obtained in Example 1 was dissolved in 2 ml of acetic anhydride and stirred at room temperature for 1 hour. Then ether was added and hydrogen chloride gas was introduced thereto. Thus 0.9 g of 3-acetyl-6β-acetylthio-N-cyclopropylmethylnormorphine hydrochloride of the formula (VII) was obtained in the form of colorless crystals. (m.p.: >200° C, slowly decomposed).

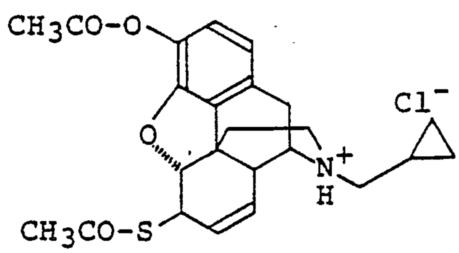

(VII)

Optical rotation $[\alpha]_D^{20}$ : -260.2° ($H_2O$, C = 0.327)
Elemental analysis as $C_{24}H_{27}NO_4S \cdot HCl$
(molecular weight: 462.007)

| | | | |
|---|---|---|---|
| calculated (%): | C 62.39; | H 6.11; | N 3.03 |
| found (%): | C 62.12; | H 6.25; | N 2.87 |

The 3-acetyl-6β-acetylthio-N-cyclopropylmethylnormorphine obtained in Example 1 was dissolved in either and hydrogen chloride gas was introduced thereto. The hydrochloride thus obtained showed the same properties as those shown above.

Example 3

Starting from 1.4 g of N-allylnormorphine, the procedure of Example 1 or 2 was repeated. Thus 0.9 g of 3-acetyl-6β-acetylthio-N-allylnormorphine hydrochloride of the formula (VIII) was obtained (m.p.: 207 - 210° C).

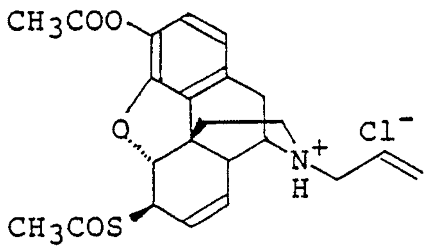

(VIII)

Optical rotation $[\alpha]_D^{25}$ : -272.6° ($H_2O$, C = 0.171)
Elemental analysis as $C_{23}H_{25}NO_4S \cdot HCl \cdot H_2O$
(molecular weight: 465.996)

| | | | |
|---|---|---|---|
| calculated (%): | C 59.28; | H 5.84; | N 3.00 |
| found (%): | C 59.33; | H 6.07; | N 2.96 |

Example 4

1.6 g of the 6β-acetylthio-N-cyclopropylmethylnormorphine obtained in Example 1 was added to 50 ml of a 0.2 N potassium hydroxide solution in ethanol. The obtained mixture was stirred under a nitrogen gas stream at room temperature for 30 minutes. The reaction mixture was then poured into a saturated aqueous solution of ammonium chloride and extracted with chloroform. The extract was dehydrated and concentrated under reduced pressure. Thus 6β-mercapto-N-cyclopropylmethylnormorphine was obtained. This product was reacted with 0.88 ml of propionyl chloride in chloroform in the presence of 1.4 ml of triethylamine. After 2 hours, the reaction mixture was washed with a saturated aqueous solution of sodium hydrogencarbonate and a saturated aqueous solution of common salt, dehydrated and concentrated under reduced pressure. The residue was purified by silica gel column chromatography (3% methanol/chloroform). Thus 1.2 g of 3-propionyl-6β-propionylthio-cyclopropylmethylnormorphine was obtained [mass spectrum (m/z): 453 (M)]. This product was converted into its hydrochloride of the formula (IX) in a conventional manner (m.p.: 202 - 206° C).

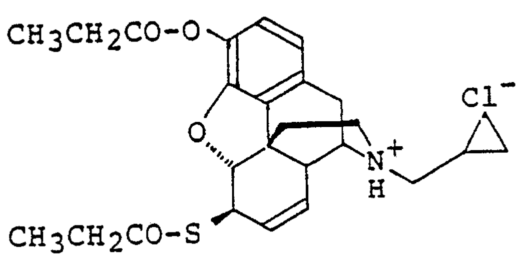

(IX)

Optical rotation $[\alpha]_D^{25}$ : -252.88° (MeOH, C = 0.200)

Example 5

The procedure of Example 4 was repeated except that the propionyl chloride was replaced by isobutyl chloride. Thus 3-isobutylyl-6β-isobutylylthio-cyclopropylmethylnormorphine hydrochloride of the formula (X) was obtained at a yield of 75% (m.p.: 210 - 215° C).

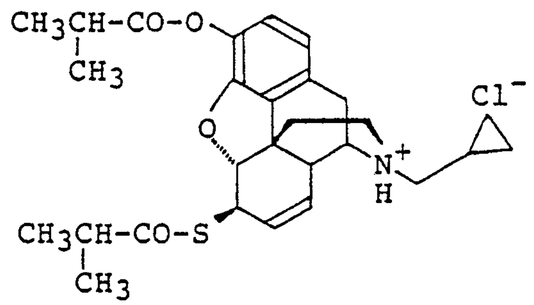

(X)

Optical rotation $[\alpha]_D^{25}$ : -262.62° (MeOH, C = 0.198)

Example 6 Analgesic Examination by Radiant Heat-Stimulation Test:

The compounds of the present invention and morphine hydrochloride were subcutaneously injected into male mice (Slc: ddy) aged 4 weeks. Each group had 7 animals. An hour after the injection, the analgesic effect of each compound was examined by radiant heat-stimulation test [refer to A.G. Hayes, M.J. Sheehan, M.B. Tyers; Brit. J. Pharmacol., 91, 111 - 115 (1987)]. Namely, the tail of each mouse was irradiated with intense light and the time required until the animal switched its tail was measured. The value thus measured was referred to as the pain threshold. Fig. 1 shows the results.

The compounds (VI) and (VII) showed each an analgesic effect depending on dose within a range of from 0.03 to 1.0 mg/kg. The effects of both of these compounds were statistically significant at a dose of 1.0 mg/kg ($p < 0.01$ in Dunnett's t-test).

On the other hand, the morphine hydrochloride employed as a control showed a significant analgesic effect at a dose of 3.2 mg/kg or above.

Example 7 Transmural Electric Stimulation Test on an Extirpated Guinea Pig Ileum Piece:

The effects of the compounds were examined by applying transmural electric stimulation (0.1 Hz) to extirpated guinea pig ileum pieces while using the smooth muscle contraction as an indication [refer to H.K. Kopsterlitz, A.A. Waterfield; Annu. Rev. Pharmacol., 15, 29 - 47 (1975)]. Table 1 shows the results.

The data shown in Table 1 were calculated according to a method reported by Arunlakshana and Schild [refer to 0. Arunlakashana, H.O. Schild; Brit. J. Pharmacol., 14, 48 -58 (1959)]. Each value is expressed in mean ± standard deviation.

Table 1

| Transmural Electric Stimulation Test on an Extirpated Guinea Pig Ileum Piece | | | |
|---|---|---|---|
| | $pD_2$ | $pA_2$ (naloxone) | Slope |
| Morphine hydrochloride | 7.32 ± 0.13 | 8.45 ± 0.13 | (1.03 ± 0.09) |
| Compound VI | 8.38 ± 0.06 | 7.75 ± 0.12 | (0.93 ± 0.09) |
| Compound VII | 9.01 ± 0.16 | 7.80 ± 0.13 | (0.84 ± 0.11) |

Example 8 Analgesic Effect Examined by Acetic Acid-Writhing Test:

Male mice (Slc: ddy) aged 5 weeks were divided into groups each having 7 animals. The compounds of the present invention and morphine hydrochloride and pentazocine, which were employed as control compounds, were subcutaneously injected into these animals. After 30 minutes, 0.6% acetic acid was intraperitoneally administered to each mouse in a dose of 0.1 ml/10 g. 10 minutes thereafter, the writhings thus induced were counted within 10 minutes. Table 2 shows the results expressed in $ED_{50}$. The acetic acid-writhing test was conducted according to a method reported by R. Koster, M. Anderson and E.I. Debeer [refer to Fed. Proc., 18, 412 (1959)].

Table 2

| Analgesic Effects on Mice in the Acetic Acid-Writhing Test | |
|---|---|
| Compound | $ED_{50}$ (mg/kg) |
| Morphine hydrochloride | 0.139 |
| Pentazocine | 0.869 |
| Compound VII | 0.062 |
| Compound IX | 0.047 |

## Claims

1. A compound represented by the following general formula:

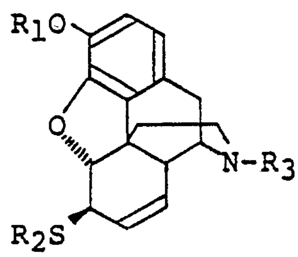

wherein $R_1$ represents a hydrogen atom or a lower alkanoyl group;
$R_2$ represents a lower alkanoyl group; and
$R_3$ represents a cyclopropylmethyl or an allyl group),
and a pharmaceutically acceptable acid addition salt thereof.

2. A pharmaceutical composition which comprises a compound represented by the following general formula:

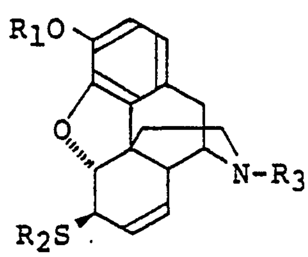

wherein $R_1$ represents a hydrogen atom or a lower alkanoyl group;
$R_2$ represents a lower alkanoyl group; and
$R_3$ represents a cyclopropylmethyl or an allyl group),

or a pharmaceutically acceptable acid addition salt thereof.

3. The pharmaceutical composition according to Claim 2 which is used as an analgesic.

4. The pharmaceutical composition according to Claim 2 which is used as an anesthetic.

*Fig. 1*

ANALGESIC EXAMINATION ON MICE BY RADIANT HEAT-STIMULATION TEST

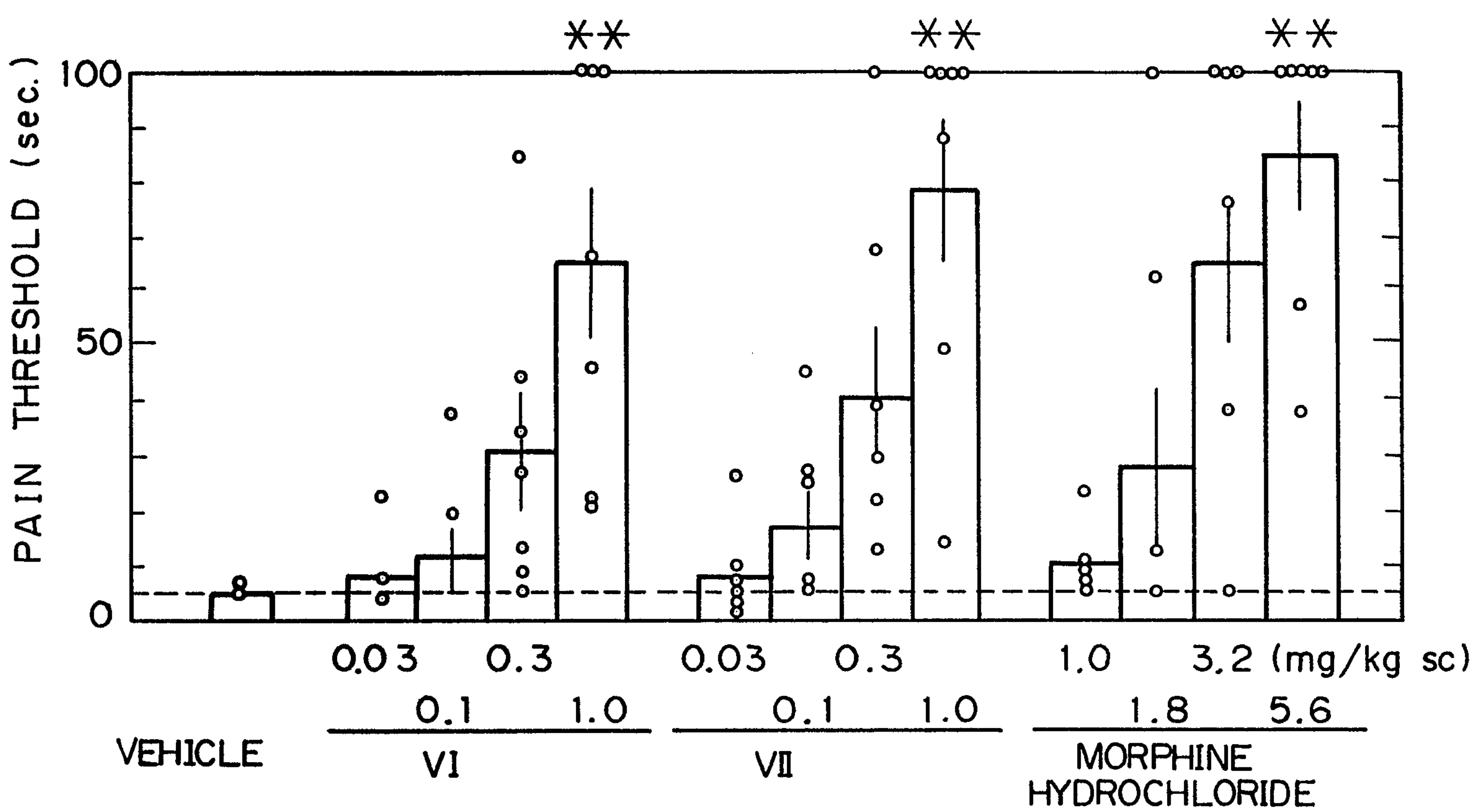


(MEAN OF 7 ANIMALS ± S.E.) **: P < 0.01 (DUNNETT'S TEST)

European Patent Office

# EUROPEAN SEARCH REPORT

Application Number

EP 89 12 3660

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | JOURNAL OF MEDICINAL CHEMISTRY vol. 7, no. 2, March 1964, pages 127-131; M. GATES et al.: "Some Potent Mosphine Antagonists Possessing High Analgesic Activity" * page 128, formula III; pages 128,129, paragraph "Pharmacological Results" * | 1-3 | C 07 D 489/00<br>A 61 K 31/485 |
| Y | CHEMICAL ABSTRACTS vol. 109, no. 21, 21 November 1988, page 49, column 1, abstract no. 183385r, Columbus, Ohio, USA; I. TAKAYANAGI et al.: "Some pharmacological properties of a newly synthesized morphine derivative (-)-6beta-acetylthiomorphine" & Arch. Int. Pharmacodyn. Ther. 1988, vol. 294, pages 71-84 | 1-3 | |
| Y | CHEMICAL AND PHARMACEUTICAL BULLETIN vol. 36, no. 6, 1988, pages 2282-2285; I. FUJII et al.: "Design and synthesis of sulfur-containing morphine and an opioid receptor probe" * pages 2282,2283, chart 1; page 2284, chart 2; page 2284, last paragraph - page 2285, line 1 * | 1-3 | TECHNICAL FIELDS SEARCHED (Int. Cl.5)<br>C 07 D 489/00 |
| A | CHEMICAL ABSTRACTS vol. 70, no. 19, 12 May 1969, page 367, column 2, abstract no. 88020k, Columbus, Ohio, USA; R. BOGNAR et al.: "Conversions of tosyl and mesyl derivatives of the morphine series. Synthesis of acetylthio and mercapto derivatives" & Acta Chem. (Budapest) 169, vol. 59, no. 1, pages 161-164 | 1 | |

The present search report has been drawn up for all claims

| Place of search | Date of completion of the search | Examiner |
|---|---|---|
| BERLIN | 23-02-1990 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document